# EUROPEAN PATENT SPECIFICATION

(11) **EP 4 232 165 B1**
(45) Date of publication and mention of the grant of the patent: **17.06.2026**
(21) Application number: 21887291.9
(22) Date of filing: 26.10.2021
(51) Int. Cl.: A62B 18/00, A61L 9/14, A61L 9/22, A42B 1/017, A42B 3/28

(54) **JET AIR CURTAIN FOR PERSONAL RESPIRATORY PROTECTION**
LUFTVORHANG FÜR DEN PERSÖNLICHEN ATEMSCHUTZ
RIDEAU D'AIR À JET POUR PROTECTION RESPIRATOIRE PERSONNELLE

(30) Priority: 26.10.2020 US 202063105499 P; 25.10.2021 US 202117509741
(43) Date of publication of application: 30.08.2023
(73) Proprietor: The Regents Of The University Of Michigan, Ann Arbor, MI 48109-2590 (US)
(72) Inventor: CLACK, Herek L., Ann Arbor, Michigan 48104 (US)
(74) Representative: Witte, Weller & Partner Patentanwälte mbB
(86) International application number: PCT/US2021/056546
(87) International publication number: WO 2022/093743

(56) References cited:
- JP-A- 2006 326 475
- JP-U- H 077 655
- KR-A- 20160 090 181
- KR-B1- 100 710 693
- KR-Y1- 200 470 938
- US-A- 2 560 215
- US-A1- 2013 118 506
- US-A1- 2018 311 515

## Description

### FIELD

The present disclosure relates to a jet air curtain for personal respiratory protection and, more particularly, relates to a Jet air curtain wearable visor for personal respiratory protection.

### BACKGROUND AND SUMMARY

This section provides background information related to the present disclosure which is not necessarily prior art. This section provides a general summary of the disclosure, and is not a comprehensive disclosure of its full scope or all of its features. Further areas of applicability will become apparent from the description provided herein.

Biological contaminants in air, such as bacteria, spores, and viruses, are classified collectively as infectious aerosols (IAs). IAs have two defining characteristics: aerosol transport and aerosol infectivity. IAs can pose threats in an array of contexts ranging from bio-terrorism to healthcare to agriculture.

High-Efficiency Particle Arresting (HEPA) filtration is a very mature, widely used approach for respiratory protection (e.g., respiratory facemasks). However, by operating solely on the principle of filtration, HEPA filters provide protection only by impeding IA transport. Respiratory protection that relies on impeding IA transport results in undesirable design and operating parameters, such as low permeability, high differential pressure and restricted air flow rates , as well as the need for perpetual filter replacement and maintaining the integrity of airtight seals. Such parameters are not insurmountable in the context of building or vehicle ventilation systems. However, for protection of an individual, accommodating these parameters becomes more difficult. For example, filtration-based respiratory protection (e.g., N95 masks) introduces substantial obstruction of airflow causing breathing restriction. Filter-based respiratory protection requires custom fitted masks to establish an initial airtight seal around the individual's breathing zone. This seal can be compromised by facial hair and extreme facial movements, exuded perspiration and skin oils, and abrupt changes in facial structure (e.g., hematomas, extreme facial expressions). Breathing resistance and mask weight both increase with use as the filter absorbs moisture from the air. Finally, activities that require physical or visual access to the nose and mouth (e.g., eating, drinking, dental procedures, lip-reading) cannot be engaged in while wearing filter-based respiratory protection.

Jet air curtain wearable device is known from documents JP 2006 326475 A and KR 2016 0090181 A. Further prior art is disclosed in US 2013/118506 A1, KR 200 470 938 Y1, US 2018/311515 A1, JP H07 7655 U, and US 2 560 215 A. A jet air curtain wearable device is defined in claim 1.

According to the principles of the present teachings, the use of jet air curtains for personal protection provides a number of advantages over existing solutions available in the art. That is, jet air curtains are well suited to protect an individual from airborne pathogens and overcome many of the disadvantages of the prior art. Firstly, a jet air curtain solution is not a mask and, thus, does not result in increased or additional burden on an individual's ability to breathe through their nose or mouth. As will be discussed herein, the jet air curtain wearable device of the present teachings protects against ambient airborne pathogens while presenting no breathing resistance to the wearer and maintains physical and visual access to the wearer's nose and mouth. Secondly, the jet air curtain wearable device of the present teachings affords the wearer protection irrespective of the presence of facial hair and does not need to physically seal to the wearer's face, which would otherwise represent a vulnerability to facial fit or inward leakage. Therefore, absent the need for a physical seal, equal protection is provided to adults and children without the need for a precise fit to each wearer; absent the potential for leakage of exhaled breath through imperfect seals, the fogging of glasses is eliminated.

Furthermore, the jet air curtain wearable device of the present teachings, which in some embodiments resembles a visor, enables wearers to easily work, speak, eat, and play as if they were wearing a baseball cap. In some embodiments, the jet air curtain wearable device of the present teachings does not obscure the wearer's face, which makes it an ideal solution for speakers, teachers, and others where non-verbal cues (such as lip movement) are critical toward comprehension--and would also be beneficial for the hearing impaired. In some embodiments, the jet air curtain wearable device of the present teachings can have a profound impact on public health by eliminating the hassles and inconveniences of wearing masks. Additionally, in some embodiments, the jet air curtain wearable device of the present teachings is a durable device that eliminates the waste associated with disposable masks.

According to the principles of the present teachings, in some embodiments, the jet air curtain wearable device can further comprise a non-thermal plasma (NTP) system operable to treat or otherwise inactivate airborne infectious agents, such as but not limited to pathogens, viruses, or other matter, contained with an input air source and output treated air as the jet air curtain, as described herein.

The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an individual wearing a jet air curtain wearable device according to some embodiments of the present teachings.
FIG. 2 is a perspective illustration of the jet air curtain wearable device according to some embodiments of the present teachings.
FIG. 3 is an enlarged illustration of an individual wearing the jet air curtain wearable device according to some embodiments of the present teachings.
FIG. 4 is a computational fluid dynamic modeling of 1 um particle trajectories about a human head (ellipsoid shape) protected by a jet air curtain visor (conical shape) wherein ambient air (with particles) flows head-on, from below at a velocity of 3.7 miles per hour, and the jet air curtain issues from the visor at 10 miles per hour.
FIG. 5 is a computational fluid dynamic modeling of 1 um particle trajectories about a human head (ellipsoid shape) protected by a sterile jet air curtain visor (conical shape), wherein ambient air (with particles) flows head-on, from below at 3.7 mph, and jet air curtain issues from visor at 47 mph.
FIG. 6 is a computational fluid dynamic modeling of 1 um particle trajectories about a human head (ellipsoid shape) protected by a sterile jet air curtain visor (conical shape) illustrating dispersion caused by the jet air curtain.
FIG. 7 is a computational fluid dynamic modeling of a jet air curtain issuing from a tabletop console showing reduction in airborne pathogen concentration in the breathing zone of subject's head (white oval). Jet diameters and initial jet velocities, clockwise from top left: 20 cm, 20 m/s; 20 cm, 2 m/s; 10 cm, 20 m/s; 10 cm, 10 m/s.
FIG. 8A is an image of energized non-thermal plasma packed bed reactor.
FIG. 8B shows concentrations of MS2 phage before and after NTP exposure (30kV AC, 170 LPM airflow rate). Results indicate viable (plaque assay) and viable+non-viable (qPCR gene copies) abundance.
FIG. 9 is a published NTP destruction efficiencies as a function of specific energy [J/L] for chemical air contaminants (gray symbols; size indicates initial concentration) compared with NTP inactivation of airborne MS2 (red symbols).
FIG. 10 is a comparison of specific energy [J/L] for NTP-based air sterilization and selected direct ozone generators.

Corresponding reference numerals indicate corresponding parts throughout the several views of the drawings.

### DETAILED DESCRIPTION

Example embodiments will now be described more fully with reference to the accompanying drawings. Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

As described herein, airborne infectious agents, such as but not limited to bacterial and viral aerosols and pathogens, have two defining characteristics- i) aerosol transport and ii) aerosol infectivity. Of these two characteristics, respiratory protective devices, such as N95/KN95 respirators, surgical masks, and cloth face coverings, act only by impeding aerosol transport. The most advanced of these devices, N95/KN95 respirators, have several intractable features: user discomfort due to significant breathing resistance; imperfect seals based on face shape, facial expression, or facial hair; mask deformation and moisture accumulation during prolonged use. The current COVID-19 pandemic has borne out experts' longstanding predictions of N95 respirator shortages. The present teachings identify a need for novel approaches to respiratory protection in densely occupied indoor work environments. The present teachings, unlike N95/KN95 respirators, do not rely on only one mode of action against airborne infectious agents. Further, the present teachings fill the capability gap of respiratory protection during dental procedures or while dining-contexts where respirators cannot be worn.

According to the principles of the present teachings, in some embodiments, a jet air curtain wearable device 10 is provided having advantageous construction that overcomes the limitations of the prior art. In some embodiments, the jet air curtain wearable device 10 is configured and operable to a protective, sterile airflow curtain about the face and/or around the breathing zone of a wearer (individual) to protect the wearer from environment contamination associated with airborne infectious agents. In some embodiments, the jet air curtain wearable device 10 is sized (small) to be integrated into wearable accessories, such as but not limited to goggles, glasses, hats, or visors. Unlike conventional facemasks and powered air-purifying respirators (PAPRs) that operate solely by deep filtration of airborne pathogens, the jet air curtain wearable device 10 of the present teachings simultaneously removes and inactivates airborne pathogens without HEPA filters.

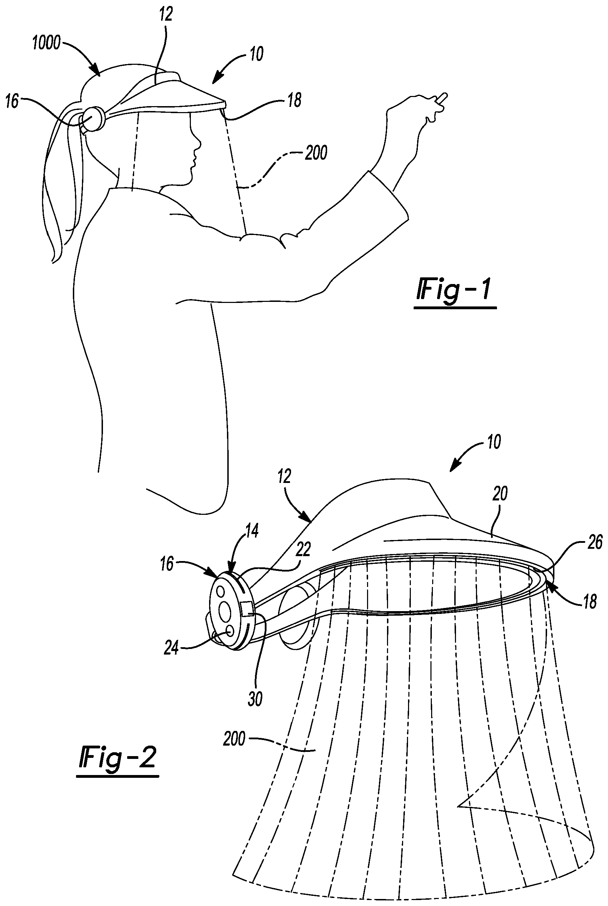

With reference to the figures, in some embodiments, jet air curtain wearable device 10 can be used by a wearer, user, or individual 1000. In some embodiments, as illustrated in FIGS. 1-3, jet air curtain wearable device 10 can be constructed in the form of a hat or visor. However, it should be understood that jet air curtain wearable device 10 can be constructed in any form that positioned a jet air curtain 200 about the face and/or around the breathing zone of the wearer 1000. The breathing zone, in some embodiments, can be that zone or volume containing air that is inhaled by the wearer 1000 during inhalation. It should be understood that jet air curtain 200 can generally be described as an airflow layer that generally prevents air outside (relative to the wearer) from generally transcending the airflow layer within the breathing zone of the wearer. Therefore, within the breathing zone of the wearer, it should be understood that the jet air curtain 200 of the present teachings substantially prevents and/or inhibits transmigration of airborne infectious agents from a position outside to within the wearer's breathing zone therefore isolating treated air within the breathing zone from contaminated air outside the breathing zone. Accordingly, it should be understood that jet air curtain wearable device 10 is configured and operable to provide respiratory protection for the wearer by forming a curved sheet of moving air (also known as an air curtain) that is generally positioned in front of and around the wearer's face, which is particularly useful in healthcare, high density, and/or congregate workplace settings. Enveloping a subject's breathing zone with a sterile air curtain eliminates issues that are innate to N95/KN95 respirator use, such as mask fit and seal, breathing resistance, and absorption of moisture and skin oils. Furthermore, sterile jet air curtain 200 provides respiratory protection in contexts where N95/KN95 masks cannot be worn, such as during dental procedures and while dining.

The movement of this airflow (jet air curtain 200) acts to deflect aerosols present and suspended in the ambient air (i.e. airborne infectious agents). As airborne infectious agents encounter this airflow, they become entrained and are faithfully carried along with the airflow; to first order accuracy, smaller aerosols follow the airflow more faithfully than larger aerosols. Further, because ambient aerosols, including airborne pathogens such as bacteria and viruses, have no means for self-propulsion, their overall movement in the ambient atmosphere is entirely dictated by a) the force of gravity, and b) the force of drag resulting from their encounter with the airflow associated with the jet air curtain 200. As these particles are both small and not particularly dense, the dominant force dictating their movement, by far, is the drag force induced by the airflow. As a result, airborne pathogens of the size of airborne viruses and bacteria faithfully follow the airflow.

With continued reference to FIGS. 1-3, in some embodiments jet air curtain wearable device 10 comprises a frame system 12 wearable by wearer 1000, an inlet system 14 configured to receive ambient air, an air treatment system 16, and an outlet system 18 configured to output jet air curtain 200 as described herein. In some embodiments, frame system 12 can comprise a physical support structure configured to mount or otherwise attached jet air curtain wearable device 10 to the wearer 1000. To this end, frame system 12 can comprise a hood, hat, visor, bill, or other system configured to support jet air curtain wearable device 10 on the head of the wearer. As described herein, frame system 12 can comprise glasses or any other structure suitable for supporting and permitting formation of jet air curtain 200. In some embodiments, frame system 12 is fastened to or worn by the wearer 1000 via a strap, hook-and-loop fastener, fabric, elastic, or other means.

In some embodiments, frame system 12 is configured to position outlet system 18 in a predetermined orientation such that output air from outlet system 18 distributed as jet air curtain 200 having a generally continuous curtain formation to safely contain the wearer's breathing zone. To this end, in some embodiments, frame system 12 can comprise an outwardly directed portion 20 offset from the wearer's face to permit the jet air curtain 200 to be formed a predetermined distance A from the wearer's face. In some embodiments, the outwardly directed portion 20 can comprise the bill or brim of a hat or visor, as illustrated.

In some embodiments, inlet system 14 can comprise an inlet orifice 22 configured to receive or intake ambient air into air treatment system 16. In some embodiments, inlet system 14 and/or air treatment system 16 can comprise an intake pump, fan, or other means to receive ambient air and treat the ambient air in the air treatment system 16, as will be discussed herein. In some embodiments, inlet system 14 and/or air treatment system 16 can comprise a system configured to provide non-hazardous air at sufficiently high pressures and volumetric flow rates to form jet air curtain 200. In some embodiments, inlet system 14 and/or air treatment system 16 can be contained within a single device wearable on the user's head, which incorporates a pump or fan for inducing the necessary airflows, along with a process for removing or otherwise neutralizing airborne contaminants in ambient air. In some embodiments, inlet system 14 and/or air treatment system 16 can be disposed as a separate unit worn elsewhere on the wearer's body and connected to the frame system 12 via an umbilical.

In some embodiments, inlet system 14 and/or air treatment system 16 can comprise a control system 24 for controlling the operation of inlet system 14, air treatment system 16, and/or outlet system 18.

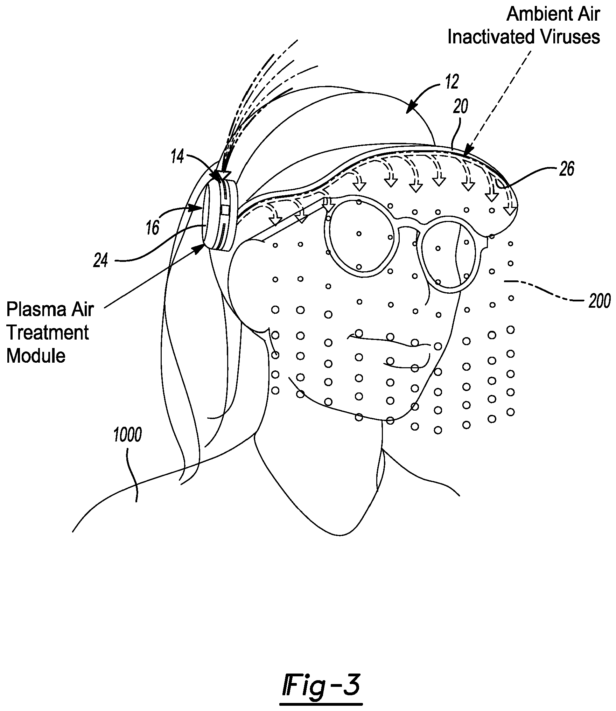

In some embodiments, air treatment system 16 is configured to treat the ambient air received from inlet system 14 prior to transport of the treated air to outlet system 18 to be dispensed as jet air curtain 200. As illustrated in FIGS. 2 and 3, outlet system 18 can comprise an outlet orifice 26 for outputting treated air, which can comprise an elongated orifice (as illustrated) and/or one or a plurality of nozzles extending continuously from a position at a first side of the wearer's head (i.e. temple region) to a position at a second side of the wearer's head (i.e. temple region); the first side being opposite the second side to form a continuous curtain extending and surrounding the wearer face. The outwardly directed portion 20 and outlet orifice 26 can be designed and oriented such that the jet air curtain 200 extends around either side of the wearer's face, terminating at the surface of the wearer's head such that there is as nearly as is feasible, an unbroken curved expanse of flowing air extending generally from ear to ear on the wearer, providing unbroken fluid dynamic protection from ambient airborne pathogens.

It should be understood that the shape, position, and operation of jet air curtain 200 significantly enhances the performance and protection thereof. That is, in some embodiments, having the jet air curtain 200 flowing downward reduces the chance that entrained aerosols are directed into the wearer's (generally downward-oriented) nostrils. The size, shape, thickness, and/or orientation of jet air curtain 200 can prevent, or at least minimize, intrusion of ambient aerosols into the breathing zone of the wearer. Such intrusion can be driven not only by ambient air currents with sufficient momentum to enter the wearer's protected breathing zone owing to the momentum that such air currents possess themselves, but also such intrusion can occur as a result of pumping and suction from within the protected breathing zone, such pumping or suction action occurring in response to the presence of the confined airflow of the jet air curtain 200. Such pumping action can occur in the presence of jets and wakes and is used to positive effect by devices known as ejectors.

It should further be understood that the jet air curtain wearable device 10 further increases the performance of jet air curtain 200 for personal respiratory protection by harnessing the interaction between the jet air curtain 200 and the torso of the wearer. As noted previously, as a result of the action of a jet, regions of lower pressure can form which, in interacting with nearby regions of higher ambient pressure, lead to pumping or suction of fluid toward the regions of low pressure. Such suction or pumping, if not addressed, could promote introduction of ambient air and suspended aerosols into the protected breathing zone. The jet air curtain 200 concept prevents such contamination of the protected breathing zone-specifically at distal locations of the jet furthest away from jet origin (i.e. outlet orifice 26)-by designing the jet air curtain 200 velocity and configuration in a way to insure that the jet impinges against the torso of the wearer. In this way, the torso of the wearer provides a solid boundary and closure of the protected breathing zone that prevents, for example, recirculation of ambient, contaminated air into the protected breathing zone through what might otherwise be the open end of the jet air curtain 200. By this same reasoning, jet air curtain wearable device 10 itself is an important component in establishing the protected breathing zone for the wearer in that the visor configuration, for example, serves as a solid boundary, prevents contaminated ambient air and suspended aerosols from being sucked or pumped by the action of the jet air curtain 200 from regions above the wearer down into the protected breathing zone.

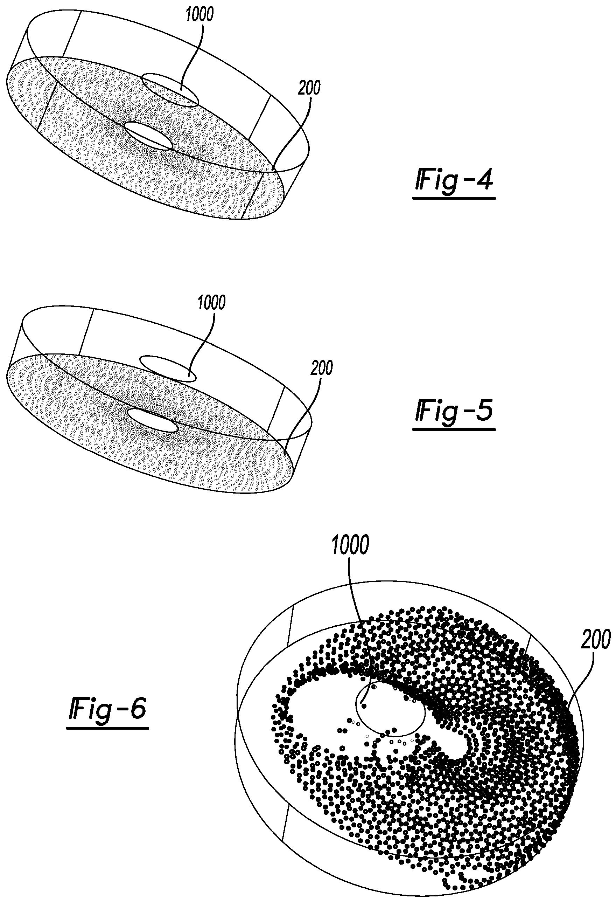

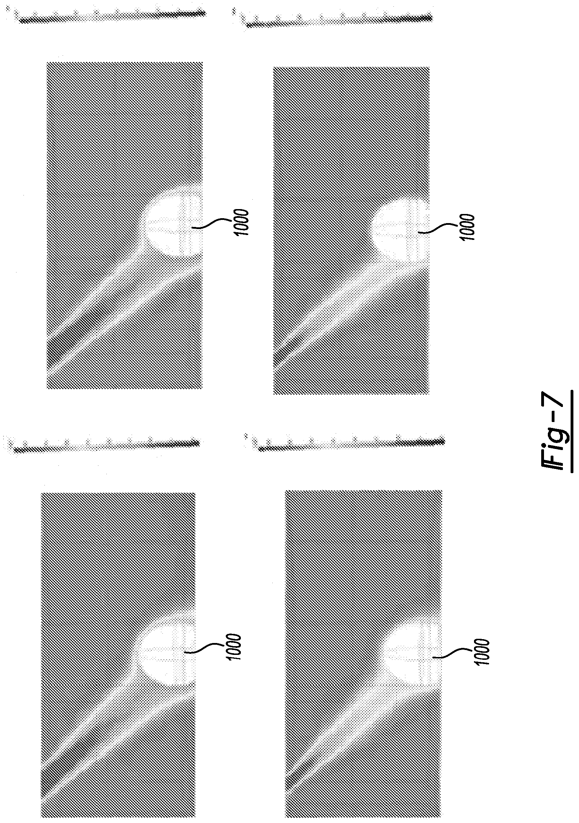

With particular reference to FIGS. 4 and 5, computational fluid dynamic (CFD) simulations have demonstrated in detail the dispersion and deflection of collections of 1 micrometer sized aerosols in a 1 or 3 m/s ambient outdoor air current air as they encounter a generic jet air curtain 200 emanating from a visor outfitted with a downward-oriented slot nozzle positioned above the eyes on an ovoid shape designed to represent a human head. Air velocities issuing from the slot nozzle were varied from 0.3 to 10 m/s and all results showed highly effective dispersion and deflection of particles sized similar to typical airborne pathogens, such as viruses and bacteria. Additionally, as illustrated in FIG. 7, the jet air curtain concept is illustrated using numerical simulation results, wherein an under-expanded jet of sterile air provides a barrier against ambient airborne pathogens (shaded area) by enveloping the breathing zone around the wearer's head (light area).

In some embodiments, air treatment system 16 can comprise systems to address, at least, aerosol infectivity. To this end, in some embodiments, air treatment system 16 can comprise a non-thermal plasma system 30. In some embodiments, non-thermal plasma system 30 can comprise a miniaturized non-thermal plasma reactor that quietly and unobtrusively supplies the protective, sterile airflow curtain of jet air curtain 200 about the face or around the breathing zone from a platform small enough to be integrated into wearable accessories, such as goggles, glasses, or visors. Unlike conventional facemasks and PAPRs that operate solely by deep filtration of airborne pathogens, non-thermal plasma system 30 simultaneously removes and inactivates airborne pathogens without HEPA filters.

Non-thermal plasmas (NTPs) are stable electrical discharges that address both aerosol transport and aerosol infectivity. As a technology platform, by operating on two protective principles (aerosol transport and aerosol infectivity), NTPs can strike a flexible and balanced approach to respiratory protection. When implemented in the form of a sterile jet air curtain 200, according to the principles of the present teaching, that envelopes an individual's breathing zone, NTPs avoid all the undesirable features of N95/KN95 respirators.

Generally, with regard to aerosol infectivity, NTPs in air produce charged and reactive radicals (RRs), mostly reactive oxygen and nitrogen species (RONS) such as O•, OH-, OH• that are orders of magnitude more reactive than ozone (Os). The RRs and RONs vigorously attack biological membranes and proteinaceous capsids, ultimately inactivating the airborne pathogen or other airborne infectious agents. During operation, NTPs charge and electrostatically remove larger (> 1 micrometer in diameter) infectious aerosols, such as respiratory droplets, from an air stream. Smaller infectious aerosols (< 1 micrometer in diameter) that remain in the air stream are inactivated by direct exposure to the reactive oxidants that vigorously attack biological membranes of bacteria and proteinaceous capsids of viruses.

Earlier generations of NTPs used to destroy chemical air contaminants suffered from high power demands, but our experiments with biological air contaminants show comparable efficacy achieved at 1/10th to 1/100th of the power required for chemical air contaminants, suggesting that inactivation of pathogens is not proscribed by the same kinetic and thermodynamic limits that dictate chemical reaction equilibrium.

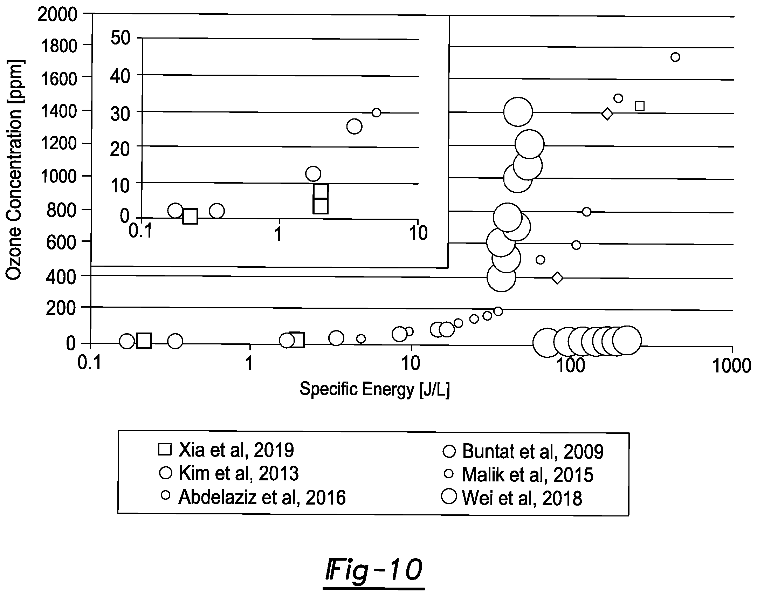

Ozone production has also been a question for NTPs used to treat ventilation air. We have found that by installing a common laser printer ozone filter, NTPs reduced residual ozone by 1 to 2 orders of magnitude, depending on airflow rate, while adding only 20 Pa to overall ΔP. In some cases, a single layer ozone filter alone was able to reduce residual ozone concentrations below the 50 ppb allowable limit for indoor air cleaners set by the California Air Resources Board (CARB). Chemical reaction engineering principles dictate that O₃ concentrations can be reduced further by increasing the ozone filter space velocity (i.e., ratio of surface area to volumetric flow rate), for example, by using a double filter layer. FIG. 10 places into perspective the ozone generation potential of NTPs used for biological applications. For five studies of direct ozone generators that used air as a feedstock, specific energy requirements (J/L) are up to 3 orders of magnitude greater than those we demonstrated for air sterilization. As noted previously, it is apparent that the NTP operating regime for biological applications deviates dramatically from that of chemical applications, on which most critiques of the former have been erroneously based.

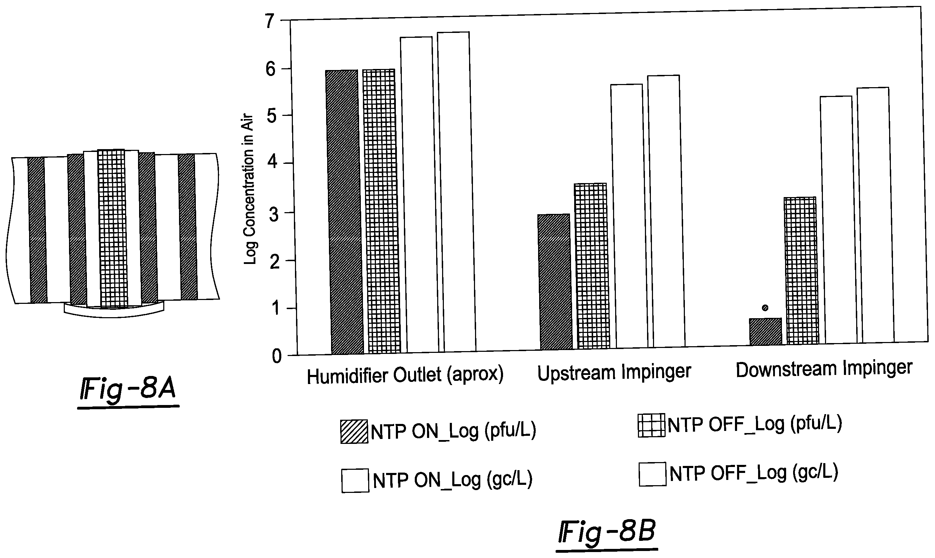

Furthermore, it has been found that air stream exposure of less than 400 milliseconds to an NTP reduced the abundance of infectious bacteriophage MS2 by more than 2.3 log at a flow rate of 170 LPM. In fact, MS2 inactivation was so thorough that viable virus concentrations were undetectable (FIGS. 8A and 8B), i.e., measured PFU/ml after NTP exposure fell below the limit of quantification. It has further been found that the same NTP process achieved comparable inactivation in air of the highly contagious PRRS virus that causes porcine reproductive and respiratory syndrome (PRRS). These results demonstrate for the first time an airborne virus known to cause human or animal disease inactivated by NTP.

Generally, with regard to aerosol transport, it is understood that jetted air directed downwardly, even in dense seating arrangements of commercial aircraft, is useful in preventing transport and/or transmission of infection. However, by design, aircraft cabin air handling systems provide pressurized airflows sufficient to withstand the drop in pressure (ΔP) caused by HEPA filters (not NTPs) and still produce sufficient momentum to form a jet of air at the nozzle exit. In contrast, traditional indoor air cleaners cannot pressurize ambient air to the same degree, and thus after the ΔP of HEPA filtration the air stream has low momentum. By comparison, the much more permeable packed bed of the NTP reactor of air treatment system 16 imposes < 45 Pa (< 0.2 in. H2O) of ΔP at an airflow rate of 170 liters per minute (LPM). Such low flow obstruction combined with rapid inactivation (< 400 milliseconds NTP exposure) and low power consumption (23 W) are the performance characteristics needed for compact, portable or distributed, filterless respiratory protection from airborne pathogens in ambient air.

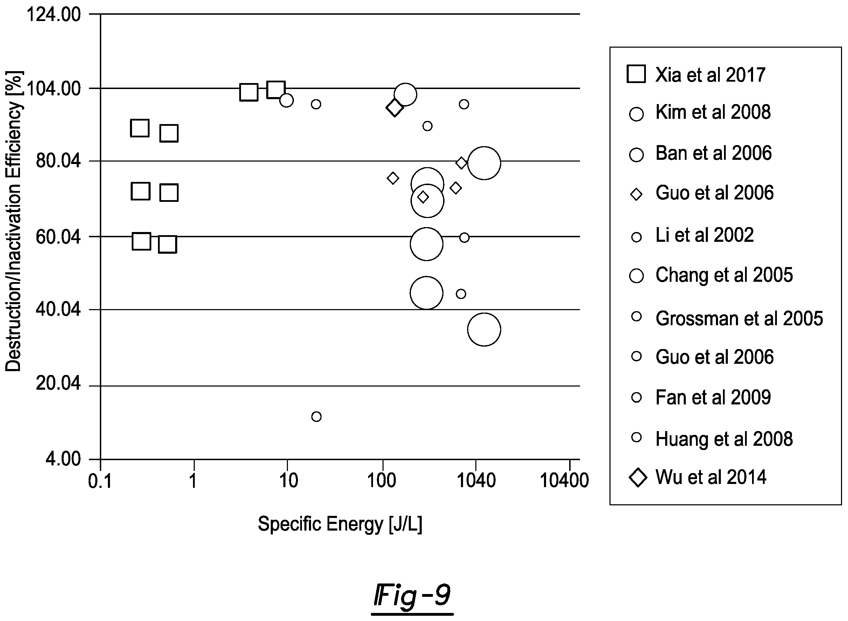

NTPs used in chemical synthesis applications have been criticized for having high power consumption. However, NTPs used for biological applications appear to operate in a different regime. FIG. 9 compares NTP process efficiency (%) for nine chemical synthesis and two biological inactivation studies as a function of specific energy consumption (J/L). The comparison shows that NTP-based airborne pathogen inactivation requires 10 to 1000 times less energy per liter of air than an array of NTP-based chemical synthesis processes. We hypothesize that partial oxidation of viral surface proteins disrupts attachment and binding with host cell receptors, preventing host cell penetration. This disruption is more facile and less constrained by thermodynamic limits on chemical reactions.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment. The same may also be varied in many ways. Such variations are not to be regarded as a departure from the disclosure, and all such modifications are intended to be included within the scope of the disclosure which is defined by the appended claims.

## Claims

1. A jet air curtain wearable device (10) that is wearable by an individual (1000) and configured to create a jet air curtain (200) generally containing a breathing zone of the individual, the breathing zone being a zone or volume containing air that is inhaled by the individual during inhalation, the jet air curtain wearable device (10) comprising:
a frame system (12) comprising a visor or a hat configured to be worn on a head of the individual;
an air treatment system (16) configured to receive the ambient air from an inlet system (14) mounted directly on the air treatment system and configured to receive ambient air, the air treatment system comprises a system for pumping the ambient air;
an outlet system (18) supported by the frame system, the outlet system operably coupled with the air treatment system and configured to receive the treated air and output the treated air directly from an outlet orifice (26) formed in a distal end of a brim of the visor or hat as a jet air curtain generally surrounding the breathing zone of the individual,
**characterized in that**
the air treatment system (16) is mounted directly to the frame system (12) and includes a non-thermal plasma reactor configured to receive the ambient air and subject the ambient air to a sterilizing condition and output the treated air.

2. The jet air curtain wearable device according to Claim 1 wherein the non-thermal plasma reactor is configured to output an electrical discharge operable to produce charged and reactive radicals to inactivate airborne infectious agents.

3. The jet air curtain wearable device according to Claim 1 wherein the non-thermal plasma reactor is configured to remove infectious aerosols greater than 1 micrometer in diameter from the ambient air.

4. The jet air curtain wearable device according to Claim 1 wherein the non-thermal plasma reactor is configured to inactivate infectious aerosols less than 1 micrometer in diameter in the ambient air.

5. The jet air curtain wearable device according to Claim 1 wherein the outlet system comprises the outlet orifice (26) configured to output the treated air as the jet air curtain in a downward direction.

6. The jet air curtain wearable device according to Claim 5 wherein the jet air curtain has a flowrate above 50 liters per minute.

7. The jet air curtain wearable device according to Claim 5 wherein the outlet orifice comprises a plurality of nozzles.

8. The jet air curtain wearable device according to Claim 5 wherein the outlet orifice comprises a continuously opened orifice extending from a first side of a head of the individual to an opposing side of the head of the individual.

9. The jet air curtain wearable device according to Claim 5 wherein the outlet orifice is configured such that the jet air curtain generally surrounding the breathing zone of the individual is bounded on top by the frame system, on sides by a head of the individual, and on bottom by a torso of the individual.

## Patentansprüche

1. Tragbare Düsenluftschleier-Vorrichtung (10), die von einer Person (1000) tragbar und konfiguriert ist, um einen Düsenluftschleier (200) zu erzeugen, der im Allgemeinen eine Atmungszone der Person umschließt, wobei die Atmungszone eine Zone oder ein Volumen ist, das Luft enthält, die von der Person während der Inhalation eingeatmet wird, wobei die tragbare Düsenluftschleier-Vorrichtung (10) umfasst:
ein Rahmensystem (12), das einen Schirm oder einen Hut umfasst, der konfiguriert ist, um auf einem Kopf der Person getragen zu werden;
ein Luftbehandlungssystem (16), das konfiguriert ist, um die Umgebungsluft von einem Einlasssystem (14) zu empfangen, das direkt an dem Luftbehandlungssystem montiert und konfiguriert ist, um Umgebungsluft zu empfangen, wobei das Luftbehandlungssystem ein System zum Pumpen der Umgebungsluft umfasst;
ein Auslasssystem (18), das von dem Rahmensystem getragen wird, wobei das Auslasssystem mit dem Luftbehandlungssystem wirkverbunden und konfiguriert ist, um die behandelte Luft zu empfangen und die behandelte Luft direkt aus einer Auslassöffnung (26) auszugeben, die in einem distalen Ende einer Krempe des Schirms oder Huts ausgebildet ist, als ein Düsenluftschleier, der die Atmungszone der Person im Allgemeinen umgibt,
**dadurch gekennzeichnet, dass**
das Luftbehandlungssystem (16) direkt an dem Rahmensystem (12) montiert ist und einen nicht-thermischen Plasmareaktor einschließt, der konfiguriert ist, um die Umgebungsluft zu empfangen und die Umgebungsluft einer sterilisierenden Bedingung auszusetzen und die behandelte Luft auszugeben.

2. Tragbare Düsenluftschleier-Vorrichtung nach Anspruch 1, wobei der nicht-thermische Plasmareaktor konfiguriert ist, um eine elektrische Entladung auszugeben, die geeignet ist, geladene und reaktive Radikale zu erzeugen, um luftgetragene infektiöse Agenzien zu inaktivieren.

3. Tragbare Düsenluftschleier-Vorrichtung nach Anspruch 1, wobei der nicht-thermische Plasmareaktor konfiguriert ist, um infektiöse Aerosole mit einem Durchmesser von mehr als 1 Mikrometer aus der Umgebungsluft zu entfernen.

4. Tragbare Düsenluftschleier-Vorrichtung nach Anspruch 1, wobei der nicht-thermische Plasmareaktor konfiguriert ist, um infektiöse Aerosole mit einem Durchmesser von weniger als 1 Mikrometer in der Umgebungsluft zu inaktivieren.

5. Tragbare Düsenluftschleier-Vorrichtung nach Anspruch 1, wobei das Auslasssystem die Auslassöffnung (26) umfasst, die konfiguriert ist, um die behandelte Luft als den Düsenluftschleier in einer nach unten gerichteten Richtung auszugeben.

6. Tragbare Düsenluftschleier-Vorrichtung nach Anspruch 5, wobei der Düsenluftschleier eine Durchflussrate von über 50 Litern pro Minute aufweist.

7. Tragbare Düsenluftschleier-Vorrichtung nach Anspruch 5, wobei die Auslassöffnung eine Vielzahl von Düsen umfasst.

8. Tragbare Düsenluftschleier-Vorrichtung nach Anspruch 5, wobei die Auslassöffnung eine durchgehend geöffnete Öffnung umfasst, die sich von einer ersten Seite eines Kopfes der Person zu einer gegenüberliegenden Seite des Kopfes der Person erstreckt.

9. Tragbare Düsenluftschleier-Vorrichtung nach Anspruch 5, wobei die Auslassöffnung so konfiguriert ist, dass der die Atmungszone der Person im Allgemeinen umgebende Düsenluftschleier oben durch das Rahmensystem, an den Seiten durch einen Kopf der Person und unten durch einen Rumpf der Person begrenzt ist.

## Revendications

1. Dispositif portable à rideau d'air à jet (10) qui peut être porté par un individu (1000) et conçu pour créer un rideau d'air à jet (200) contenant généralement une zone de respiration de l'individu, la zone de respiration étant une zone ou un volume contenant de l'air qui est inhalé par l'individu pendant l'inhalation, le dispositif portable de rideau d'air à jet (10) comprenant :
un système de monture (12) comprenant une visière ou un chapeau conçu pour être porté sur la tête de l'individu ;
un système de traitement d'air (16) conçu pour recevoir l'air ambiant d'un système d'entrée (14) monté directement sur le système de traitement d'air et conçu pour recevoir l'air ambiant, le système de traitement d'air comprend un système pour pomper l'air ambiant ;
un système de sortie (18) supporté par le système de monture, le système de sortie étant accouplé de manière fonctionnelle au système de traitement d'air et conçu pour recevoir l'air traité et délivrer en sortie l'air traité directement depuis un orifice de sortie (26) formé dans une extrémité distale d'un bord de la visière ou du chapeau sous forme d'un rideau d'air à jet entourant généralement la zone de respiration de l'individu,
**caractérisé en ce que**
le système de traitement d'air (16) est monté directement sur le système de monture (12) et comporte un réacteur à plasma non thermique configuré pour recevoir l'air ambiant, soumettre l'air ambiant à une condition de stérilisation et délivrer en sortie l'air traité.

2. Dispositif portable à rideau d'air à jet selon la revendication 1, dans lequel le réacteur à plasma non thermique est configuré pour délivrer en sortie une décharge électrique permettant de produire des radicaux chargés et réactifs pour inactiver des agents infectieux aéroportés.

3. Dispositif portable à rideau d'air à jet selon la revendication 1, dans lequel le réacteur à plasma non thermique est conçu pour éliminer des aérosols infectieux de plus de 1 micromètre de diamètre de l'air ambiant.

4. Dispositif portable à rideau d'air à jet selon la revendication 1, dans lequel le réacteur à plasma non thermique est conçu pour inactiver des aérosols infectieux de moins de 1 micromètre de diamètre dans l'air ambiant.

5. Dispositif portable à rideau d'air à jet selon la revendication 1, dans lequel le système de sortie comprend l'orifice de sortie (26) conçu pour délivrer en sortie l'air traité sous la forme du rideau d'air à jet dans une direction vers le bas.

6. Dispositif portable à rideau d'air à jet selon la revendication 5, dans lequel le rideau d'air à jet a un débit supérieur à 50 litres par minute.

7. Dispositif portable à rideau d'air à jet selon la revendication 5, dans lequel l'orifice de sortie comprend une pluralité de buses.

8. Dispositif portable à rideau d'air à jet selon la revendication 5, dans lequel l'orifice de sortie comprend un orifice ouvert de manière continue s'étendant d'un premier côté d'une tête de l'individu vers un côté opposé de la tête de l'individu.

9. Dispositif portable à rideau d'air à jet selon la revendication 5, dans lequel l'orifice de sortie est conçu de telle sorte que le rideau d'air à jet entourant généralement la zone de respiration de l'individu est délimité en haut par le système de monture, sur les côtés par une tête de l'individu, et en bas par un torse de l'individu.
